# EUROPEAN PATENT APPLICATION

(11) **EP 1 811 296 A1**
(43) Date of publication of application: **25.07.2007**
(21) Application number: 07008527.9
(22) Date of filing: 21.08.2001
(51) Int. Cl.: G01N 33/483, A61B 5/00, A61B 5/04, G01N 27/27, G01N 27/416, A61B 5/103, G01N 33/50

(54) **Methods and apparatus for measuring skin surface condition**

(30) Priority: 23.08.2000 JP 2000252732; 13.12.2000 JP 2000378581
(62) Divisional of application: 01956991.2
(71) Applicant: Shiseido Company, Limited, Chuo-ku Tokyo 104-8010 (JP)
(72) Inventor: Denda, Mitsuhiro, Yokohama-shi Kanagawa 236-8643 (JP); Ashida, Yutaka, Yokohama-shi Kanagawa 236-8643 (JP); Inoue, Kaori, Yokohama-shi Kanagawa 236-8643 (JP)
(74) Representative: Hollatz, Christian

(57) **Abstract**

The present invention relates to methods and apparatus for measuring the condition of skin surface, particularly a potential condition.

An object of the present invention is to provide a method and apparatus for simply measuring a potential condition of the skin surface, particularly without imparting pain to a human. In addition, another object of the present invention is to provide a method for digitalizing human senses derived from stimulation to the skin surface and a method for measuring the effect of a drug for warding off the stimulation.

Means adopted for solving the problems are to measure the potential difference between a skin section, which is floated on the surface of a culture solution, and the culture solution, and to impart stimulation to the skin or to add a drug further upon measurement in order to carry out the measurement.

From the foregoing, the potential condition of the skin surface can be measured simply, particularly without imparting pain to a human, enabling for digitalization of the condition of stimulation to the skin surface and assessment and development of a drug for warding off stimulation.

## Description

### Technical Field

The present invention relates to methods and apparatus for measuring the skin surface condition. More particularly, the present invention relates to, first, a method of making a skin model on the surface of which a skin section is floated with its surface upward, and measuring the potential condition of the skin surface from the potential difference between the skin surface and the culture solution, a method of measuring the degree of stimulation from the potential difference, which changes after a stimulating factor is imparted to the skin surface or a culture solution, and a method of measuring the degree of inhibition of the skin stimulation having changed after drug is added to the stimulated skin, as well as an apparatus which is suitable for practicing these methods.

Secondly, the present invention relates to a method of measuring the potential condition of the skin surface simply from the potential difference between the skin surface and a sublingual, without giving pain to human, and an apparatus which is suitable for practicing the method.

### Background Art

The skin surface of a human receives various kinds of stimulation from the natural world. When the skin surface receives stimulation, a symptom appears on the skin, and at the same time, an itch, a pain, a smarting pain and the like which are expressed by human senses are affected in many cases. Also in many cases, no symptom appears but only the sense is produced. Such stimulation is pleasant in some cases, but are unpleasant in many cases. For example, stimulation such as an itch is unpleasant to human. Such stimulation belongs to the category of human senses and is a nuisance because other people do not feel or see it.

On the other hand, since there has been no method for measuring the skin surface condition, it was necessary to rely on human appeal to measure the degree of senses. Accordingly, if such stimulation could be substituted by numeric values and the like, which are objective, it would be possible to share senses, which are subjective, and furthermore, if the above numerical values and the like are used, rapid development of drugs for inhibiting unpleasant stimulation can be expected.

In addition, as described above, information about the human skin surface condition is useful in the field of dermatology. In the past, upon measuring the potential condition of the human skin surface, the dermis layer and the skin which has been vigorously rubbed with a file have been used as a standard. The latter case was defective in that the potential of a standard point changes in time, and in both cases, a severe disorder was imparted to the skin due to opening in the skin a hole reaching to the dermis layer, or rubbing the skin.

In view of the aforementioned circumstance, an objective of the present invention is to provide methods and apparatus for measuring the skin surface condition, and in particular, the first objective of the present invention is to provide a method for measuring the potential condition of the skin surface , a method for measuring and digitalizing human senses caused by stimulation on the skin surface, and furthermore, where the senses have a mentally negative effect on a human, to provide a method for measuring the effect of drugs for warding off the negativity, and an apparatus used thereof.

In addition, the second objective of the present invention is to provide a method and apparatus for simply measuring potential condition of the skin surface without giving pain to the human.

### Disclosure of the Invention

In order to solve the aforementioned problems, the present inventors studied intensively, and as a result, paid attention to the skin surface potential and discovered that the aforementioned problems can be solved by making a simple skin model, in which a skin section is floated on the surface of a culture solution with its surface upward, and measuring the potential difference between the skin surface and the culture solution, utilizing the measured value, measuring the potential condition of the skin surface using the sublingual potential as a standard, and so on, and led to the completion of the present invention.

More specifically, the present invention provides methods for measuring the skin surface condition, characterized by floating a skin section, with its surface upward, on the surface of a culture solution accommodated in a container, and measuring the potential difference between the skin surface of this skin section and said culture solution. This skin surface condition may be the potential condition of the skin surface.

Furthermore, the present invention is a method of floating a skin section, with its surface upward, on the surface of a culture solution accommodated in a container, and upon measuring the potential difference between the skin surface of this skin section and said culture solution, measuring the potential difference by imparting stimulation to the skin surface to determine a difference between the measured potential difference and the potential difference when no stimulation is imparted, in order to measure the degree of skin stimulation.

In the aforementioned measurement of the degree of the skin stimulation, the stimulation may be caused by a stimulating substance or physical stimulation.

Furthermore, the present invention is a method of floating a skin section, with its surface upward, on the surface of the culture solution accommodated in a container, and upon measuring the potential difference between the skin surface of this skin section and said culture solution, measuring the potential difference by adding a stimulating substance to the culture solution to determine a difference between themeasuredpotential and the potential difference when no stimulating substance is added, in order to measure the degree of skin stimulation.

The stimulating substance most typically used upon measuring said degree of skin stimulation is a substance which generates an itch.

Moreover, the present invention is a method of floating a skin section, with its surface upward, on the surface of the culture solution accommodated in a container, and upon measuring the potential difference between the skin surface of this skin section and said culture solution, measuring the potential difference by coating the skin surface with a drug, either before or after the stimulation is imparted to the skin surface, too determine a difference between the measured potential difference and the potential difference when the skin surface is coated with no drug, in order to measure the degree of inhibition of the skin stimulation by a drug.

In addition, the present invention is a method of floating a skin section, with its surface upward, on the surface of a culture solution accommodated in a container, and upon measuring the potential difference between the skin surface of this skin section and said culture solution, measuring the potential difference by adding a drug to the culture solution, either before or after imparting stimulation to the skin surface, to determine a difference between the measured potential difference and the potential difference when no drug is added, in order to measure the degree of inhibition of skin stimulation by a drug.

In the aforementioned measurement of the degree of inhibition of skin stimulation, the stimulation may be caused by a stimulating substance or physical stimulation.

In addition, the present invention is a method of floating a skin section, with its surface upward, on a culture solution accommodated in a container, to which a stimulating substance is added, and upon measuring the potential difference between the skin surface of this skin section and said culture solution, measuring the potential difference by coating the skin surface with a drug, either before or after a stimulating substance is added to the culture solution, to determine a difference between the measured potential difference and the potential difference when the skin surface is coated with no drug, in order to measure the degree of inhibition of skin stimulation by a drug.

Furthermore, the present invention is a method of floating a skin section, with its surface upward, on the surface of a culture solution accommodated in a container, to which a stimulating substance is added, and upon measuring the potential difference between the skin surface of this skin section and said culture solution, measuring the potential difference by adding a drug to the culture solution, either before or after a stimulating substance is added to the culture solution, to determine a difference between the measured potential and the potential when no stimulating substance is added, in order to determine the degree of inhibition of skin stimulation by a drug.

The stimulating substance most typically used upon measuring said degree of inhibition of skin stimulation is a substance which generates an itch.

Furthermore, the present invention is a skin surface condition measuring apparatus comprising a culture solution, a container accommodating a skin section floating, with its surface upward, on the surface of the culture solution, a standard electrode part communicated to said culture solution , a measuring electrode part communicated to the skin surface of said skin section, and a potential difference detecting part for detecting the potential difference between said standard electrode part and said measuring electrode part.

It is preferable that said standard electrode part used in the skin surface condition measuring apparatus using a culture solution is provided with an inner tube accommodating an internal solution immersed with a standard electrode, and an outer tube accommodating an internal solution which accommodates this inner tube to form a double tube, and that a liquid junction is attached to the wall of the inner tube.

In this case, the communication between the culture solution and the standard electrode part is made by a salt bridge connected between the culture solution and the internal solution of the outer tube.

In addition, said measuring electrode part used in the skin surface condition measuring apparatus may comprise an inner tube accommodating an internal solution immersed with a measuring electrode, and an outer tube accommodating an internal solution which accommodates this inner tube to form a double tube, and a liquid junction attached to the wall of the inner tube.

In this case, the communication between the skin surface of the skin section and the measuring electrode part is made by a salt bridge connected between the skin surface of the skin section and the internal solution in the outer tube.

Said skin surface condition in the apparatus of the present invention may be the potential condition of the skin surface.

Note that it is preferable that the culture solutions used in each of the aforementioned methods and each apparatus all contain physiological saline or amino acids.

According to the methods and apparatus for measuring the skin surface condition using a culture solution, the condition of the skin surface may be measured simply by measuring the potential difference between the skin surface of the skin section and the culture solution on which a skin section is floated using a suitable apparatus. Furthermore, by measuring the change in the said potential difference by imparting a stimulating factor to the skin surface or the culture solution, stimulation may be expressed objectively without directly employing a human yet exceeding human subjectivity. In addition, the present invention may be utilized in investigating a drug which is effective to skin stimulation due to a change in the aforementioned potential difference caused by adding a drug, and which can produce a considerable effect on the investigation of new drugs, and is very useful.

Furthermore, the present invention is a method for measuring the skin surface condition, characterized by using a sublingual potential as a standard potential upon measuring of the potential condition of the skin surface.

In order to practice this measuring method, there can be adopted a method for measuring the skin surface condition from a potential difference between a standard electrode part communicating with a sublingual part and a measuring electrode part communicating with the skin surface.

Furthermore, the present invention is the skin surface condition measuring apparatus, which is provided with a standard electrode part communicating with a sublingual part, a measuring electrode part communicating with the skin surface, and a potential difference detecting part for detecting a potential difference between the standard electrode part and the measuring electrode part.

It is preferable that a standard electrode part used in this skin surface condition measuring apparatus using a sublingual potential as a standard potential is provided with an inner tube accommodating an internal solution with a standard electrode immersed therein, and an outer tube accommodating an internal solution which accommodates this inner tube to form a double tube, and a liquid junction is attached to the wall of the inner tube.

In this case, communication between the sublingual part and the standard electrode part is made by a salt bridge connecting the sublingual part and the internal solution in the outer tube of the standard electrode part.

In addition, the measuring electrode part used in the skin surface condition measuring apparatus using a sublingual potential as a standard potential may be provided with an inner tube accommodating an internal solution with a measuring electrode part immersed therein, and an outer tube accommodating an internal solution which accommodates this inner tube to form a double tube, and a liquid junction attached to the wall of the inner tube.

In this case, communication between the skin surface and the measuring electrode part is made by a salt bridge connecting the skin surface and the internal solution in the outer tube of the measuring electrode part.

The skin surface condition using said sublingual potential of the present invention as a standard potential may be a potential condition of the skin surface.

According to the skin surface condition measuring method and apparatus using a sublingual potential as a standard potential, by using a suitable apparatus to measure the potential difference between the skin surface and the sublingual part, the skin surface condition can be measured simply without causing pain to a human.

Note that, in the present invention, the concept of the skin surface condition includes, in addition to the potential condition of skin surface, various changes in condition occurring on skin surface, for example, the change in condition and the degree thereof due to stimulation, inhibition of stimulation and the like.

### Brief Description of the Drawings

Fig. 1 is a diagram showing an embodiment of the skin surface condition measuring apparatus using a culture solution according to the present invention.
Fig. 2 is a diagram showing an embodiment of the skin surface condition measuring apparatus using a sublingual potential as a standard potential according to the present invention.
Fig. 3 is a graph showing an example of the results obtained by measuring a skin surface potential difference relative to a culture solution employing the skin surface condition measuring apparatus using a culture solution according to the present invention.
Fig. 4 is a graph showing another example of the results obtained by measuring a skin surface potential difference relative to a culture solution employing the skin surface condition measuring apparatus using a culture solution according to the present invention.
Fig. 5 is a graph showing another example of the results obtained by measuring a skin surface potential difference relative to a culture solution employing the skin surface condition measuring apparatus using a culture solution according to the present invention.
Fig. 6 is a graph showing another example of the results obtained by measuring a skin surface potential difference relative to a culture solution employing the skin surface condition measuring apparatus using a culture solution according to the present invention.
Fig. 7 is a graph showing an example employing the skin surface condition measuring apparatus using a sublingual potential as a standard potential according to the present invention.
Fig. 8 is a graph showing the correlation between a skin surface potential employing the skin surface condition measuring apparatus using a sublingual potential as a standard potential according to the present invention, and a skin surface potential according to the conventional method.

### Best Mode for Carrying Out the Invention

Embodiments of the present invention will be described in detail below. First, a method and apparatus for measuring the skin surface condition using a culture solution will be explained.

This method of the present invention includes several methods. In a first method, a skin section is floated, with its surface upward, on the surface of a culture solution accommodated in a container, and a potential difference (mV) between the skin surface of this skin section and the culture solution is measured, thereby measuring the surface condition of skin.

In a second method, upon measuring the skin surface condition of the first method, stimulation is imparted further to the skin model comprising a skin section and a culture solution to measure aforementioned potential difference. This potential difference changes as stimulation is imparted. This change means that a stream of ions has occurred in the epidermis. The direction of a stream of ions differs depending on stimulation. When there is a change compared to the value of untreated skin, it is interpreted that stimulation producing mobility of ions is imparted to the epidermis. The degree of skin stimulation is measured from the changed value. Upon imparting stimulation, the place of stimulation may be on skin surface or culture solution. In the case of skin surface, examples of methods for imparting stimulation include coating skin with a stimulating substance and physical stimulation. Also, in the case of culture solution, the method used is addition of stimulating substances. Examples of these stimulating substances include neuropeptide, histamine, surfactant, organic acid, and capsaicin. Among them, neuropeptide includes substance P. In the present invention, it is particularly useful that the stimulating substance generates an itch. In addition, examples of said physical stimulation include barrier destruction by a cellophane adhesive tape and the like.

A third method is a method for measuring the degree of inhibition by addition of a drug to the stimulated skin in the second method. In the present invention, after the aforementioned skin model is stimulated, a drug is added to measure the aforementioned potential difference, and the degree of inhibition of skin stimulation by a drug is measured from the value of potential difference which changes by addition. The present method can take various methods depending on a method of drug addition. More specifically,
(1): A skin section, the surface of which has been stimulated, is floated with its surface upward, on a culture solution, a potential difference is measured by coating skin surface with a drug, either before or after imparting skin stimulation, and the degree of inhibition of skin stimulation by a drug is determined from a value of a potential difference changed by drug coating. (2): In place of coating the skin surface in (1) with a drug, a drug is added to a culture solution to measure a potential difference, and the degree of skin stimulation by a drug is measured from the value of the potential difference, which changes by drug addition to the culture solution. (3) : A skin section is floated, on a culture solution with a stimulating substance added thereto, with the surface upward, a potential difference is measured by coating a drug on the skin surface either before or after addition of a stimulating substance, and the degree of inhibition of skin stimulation by a drug is determined from a value of the potential difference changed by drug coating. (4): In place of coating the skin surface in (3) with a drug, a drug is added to a culture solution to measure a potential difference, and the degree of inhibition of skin stimulation by a drug is measured from the value of the potential difference , which changes by drug addition to the culture solution.

In addition, the aforementioned coating of a drug may be performed either before or after imparting stimulation. When the drug is coated after imparting stimulation, the effect of alleviating the stimulation can be measured, and when the drug is coated before imparting stimulation, the effect of warding off the stimulation beforehand can be measured.

The culture solution used in the measuring methods 1 through 3 may be physiological saline and an aqueous solution containing amino acids and the like. In addition, the skin section used in the measurement may be a cultured skin, a human skin obtained upon plastic surgery and the like, and a skin derived from an animal such as a hairless mouse.

When a human skin is stimulated, a symptom appears on the skin, and at the same time, an itch, a pain, a smarting pain and the like, which are expressed by human senses, are produced in many cases. Also in many cases, no symptom appears but only the sense is produced. The aforementioned 1 through 3 methods of the present invention, enable such senses, which are subjective, to be expressed by numerical values, which are objective. As a result, for example, these methods can be utilized in searching for a drug which either alleviates or inhibits stimulation without employing a human.

Next, an apparatus which is preferably used in the method using a culture solution of the present invention will be explained with reference to the drawings.

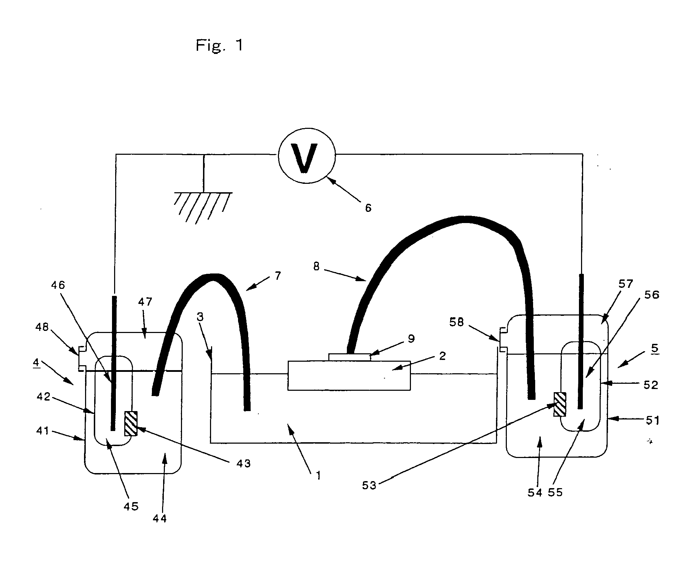

Fig. 1 shows an embodiment of the skin surface condition measuring apparatus using a culture solution in the present invention. As shown in Fig. 1, the skin surface condition measuring apparatus has a culture solution 1, a container 3 accommodating a skin section 2 floated, with its surface upward, on the surface of the culture solution, a standard electrode part 4 communicating the culture solution 1, a measuring electrode part 5 communicating to the skin surface of the skin section 2, and a potential difference detecting part 6 for detecting a potential difference between the standard electrode part 4 and the measuring electrode part 5.

The standard electrode part 4 has a double outer tube 41 and inner tube 42 which are made of a plastic, and a tube wall of the inner tube 42 has a liquid junction 43 composed of a porous ceramic having the function of not permeating a solution but permeating electrons or ions. The interiors of the double outer tube 41 and inner tube 42 are filled with internal solutions 44, 45 comprising a KCl solution having the predetermined concentration, respectively, and a standard electrode 46 comprising silver-silver chloride is immersed in the internal solution 45 of the inner tube 42. This standard electrode 46 is attached to an electrode holder 47 which also functions as a cap and closes upper ends of the double outer tube 41 and inner tube 42, and in the condition where the electrode is suspended from there via a conductor, the electrode is immersed in the internal solution 45 in the inner tube 42. A solution supplementing port 48 for supplementing the internal solution 44 in the outer tube 41 is provided on a circumferential plane near an upper end of the outer tube 41.

Note that, in the standard electrode part 4 in Fig. 1, the double outer tube 41 and inner tube 42 may all be made of glass. In addition, the liquid junction 43 used may be a known liquid junction such as a solution having a pseudo-crystal structure such as gelatin and agar, and may include a liquid junction used as a salt bridge such as a porous Bicoll glass. Alternatively, the standard electrode 46 usedmaybe an electrode comprising of calomel. In addition, the internal solution 45 used may be, other than a KC1 solution, a solution of chloride such as sodium chloride, lithium chloride and the like. The internal solution 44 in the outer tube 41 used may be, in addition to chloride used in the internal solution 45 in the inner tube 42, physiological saline.

The measuring electrode part 5 has the similar structure as that of the standard electrode part 4. More specifically, the measuring electrode part 5 has a double outer tube 51 and an inner tube 52 made of a plastic, and the tube wall of an inner tube 52 has a liquid junction 53 composed of a porous ceramic. The interiors of the double outer tube 51 and the inner tube 52 are filled with internal solutions 54 and 55, respectively, comprising of a KCl solution of a predetermined concentration, and a measuring electrode 56 composed of silver-silver chloride is immersed in the internal solution 55 of the inner tube 52. This measuring electrode 56 is attached to an electrode holder 57 which also functions as a cap to close upper ends of the double outer tube 51 and the inner tube 52, and is immersed in the internal solution 55 in the inner tube 52 in a state of being suspended from the electrode holder via conductor. A solution supplementing port 58 for supplementing the internal solution 54 in the outer tube 51 is provided on a circumferential plane near an upper end of the outer tube 51.

Note that, the measuring electrode part 5 in Fig. 1 may also take an embodiment which is similar to that of the standard electrode part 4. More specifically, the double outer tube 51 and the inner tube 52 used may be all made of glass. In addition , the liquid injunction 53 used may be the porous Bicoll glass. In addition, the aforementioned measuring electrode 56 used may comprise of calomel. In addition, the internal solution 55 used may be, other than a KC1 solution, a solution of chloride such as sodium chloride and lithium chloride. The internal solution 55 used in the outer tube 51 may be, other than chloride used in said internal solution 55, physiological saline.

Furthermore, the standard electrode part 4 in Fig. 1 is communicated to the culture solution 1 , which comprises of DMEM (Dulbecco's modified Eagle's medium), via a salt bridge 7, in which a flexible plastic tube immersed in the internal solution 44 in its outer tube 41 is filled with agarose containing physiological saline. This DMEM is a known culture solution containing amino acids and is commercially available. Other known culture solutions such as those containing physiological saline may also be used. In addition, the salt bridge 7 may be a known one, and for example, rubber tubes such as silicone and the like may be used.

In addition, the measuring electrode part 5 is communicated to the skin surface of the skin section 2 through a filter 9 placed on the skin surface via a salt bridge 8 in which a flexible plastic tube immersed in an internal solution 54 in the outer tube 51 is filled with agarose containing physiological saline. This filter 9 achieves uniformity in the contact of the salt bridge 8 with the skin surface of skin section 2. In addition, the salt bridge 8 may be a known one, and may for example use rubber tubes such as silicone and the like.

The standard electrode 46 of the aforementioned standard electrode part 4 and the measuring electrode 56 of the measuring electrode 5 are connected to a potential differential detecting part 6 to measure a potential difference thereof.

Note that, the standard electrode part used in the skin surface condition measuring apparatus may be provided with a liquid junction on the tube wall of the outer tube 41 of the standard electrode part 4 in Fig. 1. The outer tube may be provided with a solution supplementing port, which, like in Fig. 1, supplements an internal solution.

This standard electrode part has a function similar to that of the standard electrode part 4 of the skin surface condition measuring apparatus in Fig. 1, and it can be used in place of the standard electrode part 4 in Fig. 1., provided that communication is performed by immersion of this standard electrode part in the culture solution and via a liquid junction provided on an outer tube wall. Note that, embodiments of this standard electrode part are the same as those in Fig. 1.

Furthermore, the standard electrode part in the skin surface condition measuring apparatus may comprise of only the inner tube 42 of the standard electrode part 4 in Fig. 1 , namely, the inner tube 42, the liquid junction 43, the internal solution 45 and the standard electrode 46.

This standard electrode part also has a function similar to that of a standard electrode part 4 of the skin surface condition measuring apparatus in Fig. 1, and it can be used in place of the standard electrode part 4 in Fig. 1, provided that, communication with a culture solution is performed by immersion of this standard electrode part into the culture solution and via a liquid junction provided on the tube wall. In addition, embodiments in this standard electrode part are the same as in Fig. 1.

Next, a method and apparatus for measuring the skin surface condition using a sublingual potential as a standard potential will be explained.

In this method of the present invention, a sublingual area is used as a standard to measure the potential difference (mV) between the skin surface and the sublingual area. As a result, the skin surface condition is measured.

Next, an apparatus which is suitably used in the aforementioned method using a sublingual potential as a standard potential of the present invention will be explained with reference to the drawings.

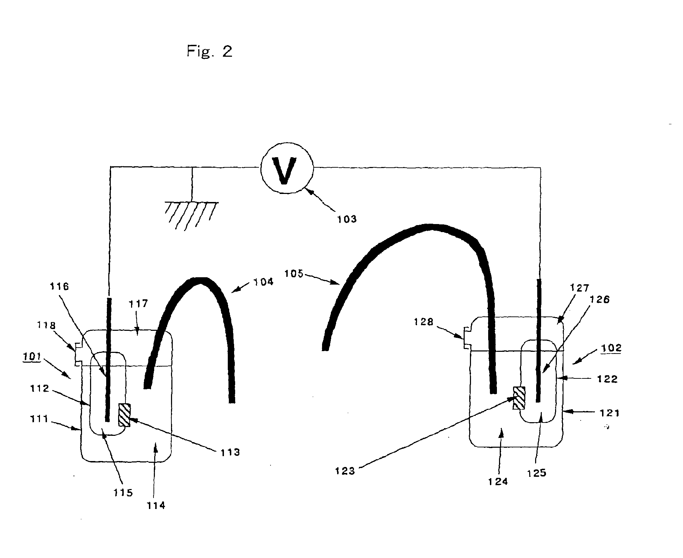

Fig. 2 shows an example of the skin surface condition (potential condition of the skin surface) using a sublingual potential as a standard potential in the present invention. As shown in Fig. 2, the skin surface condition measuring apparatus has a standard electrode part 101 communicating to a sublingual area (not shown), a measuring electrode part 102 communicating to the skin surface (not shown), and a potential difference detecting part 103 for detecting a potential difference between the standard electrode part 101 and the measuring electrode part 102.

The standard electrode part 101 has double outer tube 111 and inner tube 112 made of a plastic, and a tube wall of the inner tube 112 has a liquid junction 113 comprising a porous ceramic having the function of not permeating a solution but permeating electrons or ions. The interiors of the double outer tube 111 and inner tube 112 are filled with internal solutions 114, 115 comprising a KCl solution having the prescribed concentration, respectively, and a standard electrode 116 composed of silver-silver chloride is immersed in the internal solution 115 of the inner tube 112. This standard electrode 116 is attached to an electrode holder 117 which also functions as a cap and closes upper ends of the double outer tube 111 and inner tube 112, and in the condition where the electrode is suspended from there via a conductor, the electrode is immersed in the internal solution 115 in the inner tube 112. A solution supplementing port 118 for supplementing the internal solution 114 in the outer tube 111 is provided on a circumferential plane near an upper end of the outer tube 111.

In addition, in the standard electrode part 101 in Fig. 2, the double outer tube 111 and inner tube 112 all made of glass may be used. In addition, as the liquid junction 113, the known liquid junctions such as solutions having the pseudo-crystal structure such as gelatin, agar and the like and liquid junctions including those used as a salt bridge such as porous Bicoll glass and the like may be used. Alternatively, as the standard electrode 116, an electrode comprising calomel may be used. In addition, as the internal solution 115, a solution of chloride such as sodium chloride, lithium chloride and the like can be used in addition to a KC1 solution. As the internal solution 114 in the outer tube 111 , physiological saline may be used in addition to chloride used in the internal solution 115 in the inner tube 112.

The measuring electrode part 102 has the similar structure to that of the standard electrode part 101. That is, the measuring electrode part 102 has double outer tube 121 and inner tube 122 made of a plastic, and a tube wall of the inner tube 122 has a liquid junction 123 composed of a porous ceramic. The interiors of the double outer tube 121 and inner tube 122 are filled with internal solutions 124, 125 comprising aKCl solution having the prescribed concentration, respectively, and a measuring electrode 126 composed of silver-silver chloride is immersed in the internal solution 125 of the inner tube 122. This measuring electrode 126 is attached to an electrode holder 127 which also functions as a cap and closes upper ends of the double outer tube 121 and inner tube 122, and in the condition where the electrode is suspended from there via a conductor, the electrode is immersed in the internal solution 125 in the inner tube 122. A solution supplementing port 128 for supplementing the internal solution 124 in the outer tube 121 is provided on a circumferential plane near an upper end of the outer tube 121.

Note that the measuring electrode part 102 in Fig. 2 can also take the similar embodiment to that of the standard electrode part 101. That is, the double outer tube 121 and inner tube 122 all made of glass may be used. In addition, porous Bicoll glass may be used in the liquid junction 123. In addition, as the measuring electrode 126, an electrode comprising calomel may be used. In addition, as the internal solution 125, a solution of chloride such as sodium chloride, lithium chloride and the like can be used in addition to a KC1 solution. As the internal solution 124 in the outer tube 121, physiological saline may be used in addition to chloride used in the internal solution 125.

Furthermore, the standard electrode part 101 in Fig. 2 is communicated to a sublingual area via a salt bridge 104 immersed in the internal solution 114 in its outer tube 111. The salt bridge 104 may be the known one, and for example, flexible plastic tubes, rubber tubes such as silicone and the like, and the like which are filled with agarose containing physiological saline are used.

In addition, the measuring electrode part 102 is communicated to the skin surface via a salt bridge 105 immersed in the inner solution 124 in its outer tube 121. The salt bridge 105 may be the known one, and for example, flexible plastic tubes, rubber tubes such as silicone and the like, and the like which are filled with agarose containing physiologicalsaline are used.

The standard electrode 116 of the standard electrode 101 and the measuring electrode 126 of the measuring electrode part 102 are connected to the potential difference detecting part 103, respectively.

In order to measure the skin surface condition (potential condition) using the aforementioned apparatus, one end of a salt bridge, the other end of which is communicated to a standard electrode part, is placed in mouth of a subject to be measured, and is fixed sublingual. Upon this, one end of a salt bridge is not directly placed in mouth, but a tube such as a plastic, a silicon rubber and the like containing therein a cotton or the like containing exchangeable physiological saline is connected to an end of a salt bridge, and the tube may be placed in mouth and fixed sublingual. By doing so, since the tube can be exchanged every subject to be measured, there is no unclean feeling, and in particular, the tube is effective upon use at shop fronts and clinical places. On the other hand, after one end of a salt bridge, the other end of which is communicated to a measuring electrode part, is contacted with and fixed on the skin surface of a subject to be measured, a potential difference between the standard electrode part and the measuring electrode part is measured with a potential difference detecting part. Note that it is preferable that communication between the salt bridge and the skin surface is through a filter in order to contact both uniformly.

According to the method for measuring the skin surface condition using a sublingual potential as a standard potential, a number of information which are useful for dermatology can be obtained. For example, physical stimulation such as barrier destruction with a cellophane adhesive tape and the like is given to the skin surface, or chemical stimulation is given to the skin surface by coating a stimulating substance such as neuropeptide, histamine, surfactant, organic acid, capsaicin and the like, a potential of the skin surface having subjective sense such as itching and the like is measured, and the skin surface condition can be measured by expressing a change in the skin surface condition as an objective numerical value from comparison of potential differences before and after stimulation.

Furthermore, a potential difference is measured by coating a drug on the stimulated skin surface, and the degree of inhibition of skin stimulation by a drug may be measured from a value of a potential difference changed by drug coating.

Then, more specific Examples of the present invention will be explained.

### (Example 1)

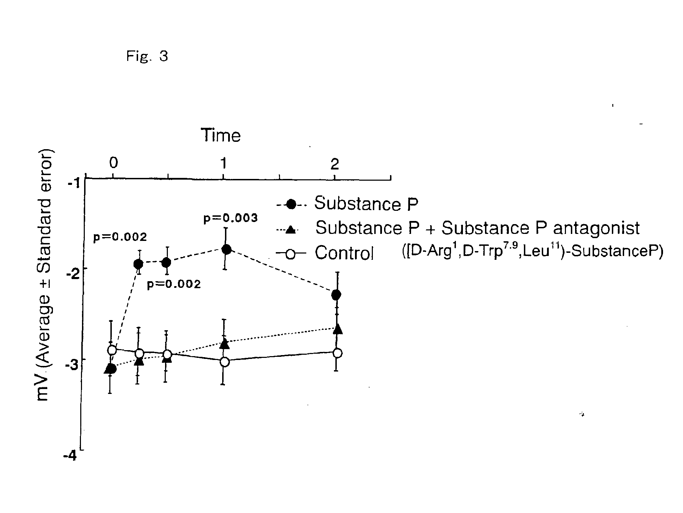

A potential difference (mV) of the skin surface relative to a culture solution was measured using the skin surface condition measuring apparatus shown in Fig. 1. The skin samples used in measurement are three kinds of skin sections of a hairless mouse, which are (1) a sample in which the skin surface is not treated, (2) a sample in which substance P (SP) of a stimulating substance is added to a culture solution, and (3) a sample in which SP antagonist ([D-Argl, D-Trp7, 9, Leull]-SubstanceP), which, together with SP, is known as a substance for warding off stimulation, is added to a culture solution. The results are shown in Fig. 3.

In Fig. 3, the horizontal axis indicates the elapsed time after addition of SP. In addition, when an SP antagonist is added, the time point of addition is indicated as zero. The vertical axis indicates potential differences. A measured value is expressed as an average of five measurements, and the fluctuation is indicated simultaneously.

As shown in Fig. 3, when SP is added to a culture solution, a rapid change in a potential difference is produced as compared with the non-treated skin surface (control). The state with a significant potential difference continues, reaching its peak 1 hour after the addition, until 2 hours after the addition when the potential difference returns to a value close to that of a control. On the other hand, when an SP antagonist is added to a culture solution before adding SP, no rapid and significant change in potential difference is produced, and a value almost the same as that of a control is indicated for 2 hours after the addition. From this, it can be seen that, when SP and an SP antagonist are added together, it can be seen that, an SP antagonist inhibits stimulation by SP, and inhibits a change in a potential difference.

### (Example 2)

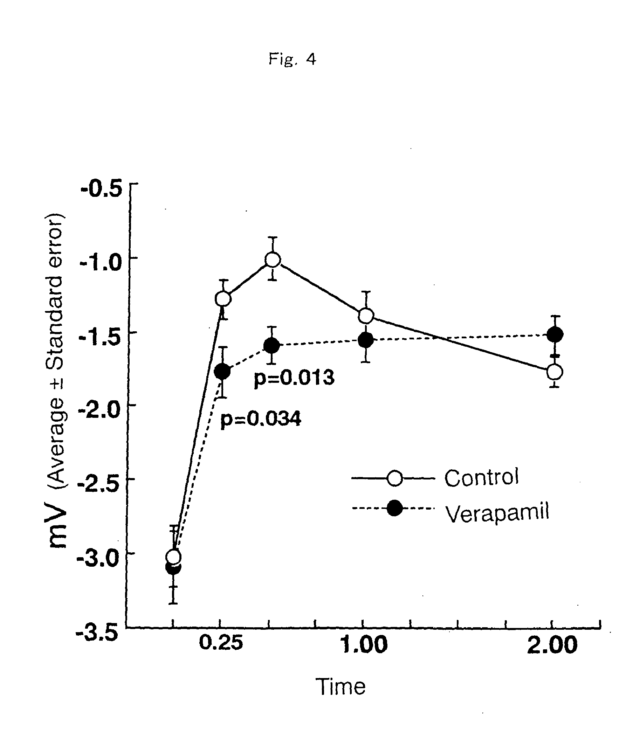

A potential difference (mV) of the skin surface relative to a culture solution was measured using the skin surface condition measuring apparatus shown in Fig. 1. The skin samples used in measurement are two kinds of skin sections of a hairless mouse, which are (1) a skin with its surface barrier-destructed with a cellophane adhesive tape (tape stripping), and (2) a skin after tape stripping, with verapamil, which is a potassium channel blocker known to function as an agent for inhibiting stimulation, added to a culture solution afterwards. The results are shown in Fig. 4.

In Fig. 4, the horizontal axis indicates the elapsed time after tape stripping. In addition, the measured value at time zero is the value of skin before applying tape stripping. In addition, when verapamil is added to a culture solution, the time of addition is indicated as zero, and the measured value at a time point zero indicates the value of skin before applying tape stripping. The vertical axis indicates potential differences. A measured value is expressed as an average of five measurements, and the fluctuation is indicated simultaneously.

As shown in Fig. 4, it can be seen that the stimulation by tape stripping produces a rapid change in potential difference as compared with before the stimulation. To the contrary, adding verapamil to a culture solution after tape stripping inhibits the change in potential difference. From this, it can be seen that, as a result of tape stripping inhibiting tape stripping stimulation, the change in potential difference is reduced.

### (Example 3)

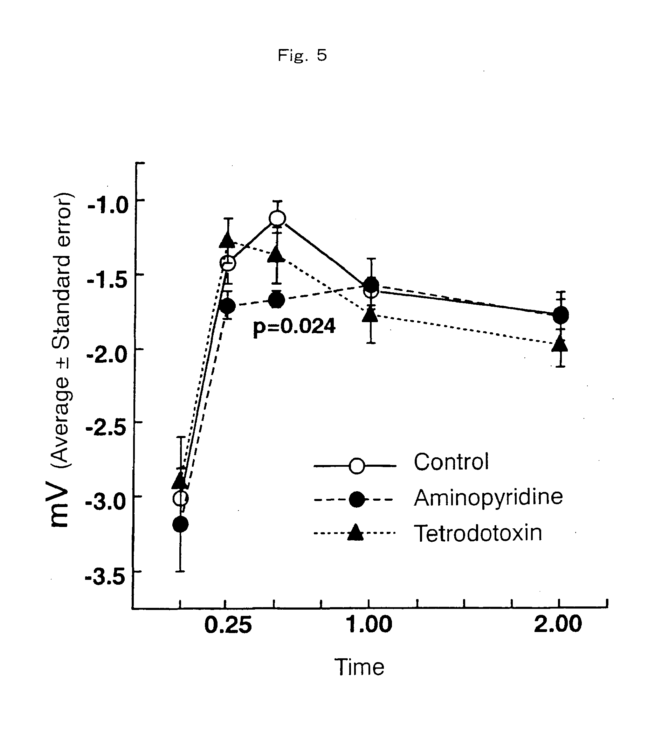

A potential difference (mV) of the skin surface relative to a culture solution was measured using the skin surface condition measuring apparatus shown in Fig. 1. The skin samples used in measurement are three kinds of skin sections of a hairless mouse, which are (1) a skin with its surface barrier-destructed with a cellophane adhesive tape (tape stripping), (2) a skin after tape stripping, with aminopyridine (AP), which is a potassium channel blocker and is known to show the stimulation inhibiting effect, added to a culture solution afterwards, and (3) a skin after tape stripping, with tetrodotoxin (TTX), which is a sodium channel blocker known not to show the stimulation inhibiting effect, added to a culture solution. The results are shown in Fig. 5.

In Fig. 5, the horizontal axis indicates the elapsed time after tape stripping. Note that the measured value at time zero is the value of skin before applying tape stripping. In addition , when AP or TTX is added to a culture solution, the time of addition is indicated as zero, and the measured value at time point zero indicates the value of skin before applying tape stripping. The vertical axis indicates potential differences. A measured value is expressed as an average of five measurements, and its fluctuation is indicated simultaneously.

As shown in Fig. 5, it can be seen that the tape stripping stimulation produces a rapid change in a potential difference as compared with before stimulation. To the contrary, adding AP to a culture solution after tape stripping inhibits the potential difference. In addition, adding TTX to a culture solution does not inhibit the change in a potential difference. From these, it can be seen that AP reduces the change in potential difference as a result of inhibiting tape stripping stimulation, and that TTX does not reduce the change in potential difference because it does not inhibit tape stripping stimulation.

### (Example 4)

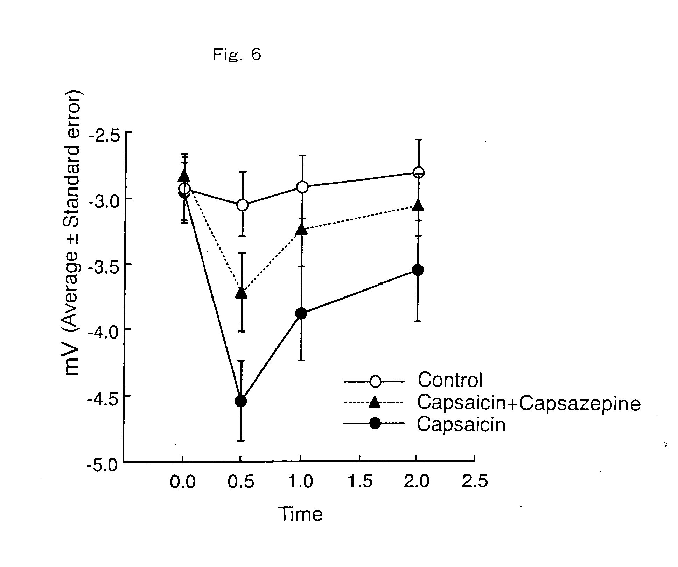

A potential difference (mV) of the skin surface relative to a culture solution was measured using the skin surface condition measuring apparatus shown in Fig. 1. The skin samples used in measurement are three kinds of skin sections of a hairless mouse, which are (1) a sample in which the skin surface is not treated, (2) a sample in which the skin surface is coated with capsaicin of a stimulating substance, and (3) a sample in which capsaicin is coated on the skin surface, and capsazepine known as a stimulation-defending substance is coated thereon afterwards. The results are shown in Fig. 6.

In Fig. 6, the horizontal axis indicates the elapsed time after coating of capsaicin. In addition, when capsazepine is coated, the time point of coating is indicated as zero. The vertical axis indicates a potential difference. The measured value is expressed as an average of five measurements, and the fluctuation is indicated simultaneously.

As shown in Fig. 6, when capsaicin is coated on skin surface, a rapid change in potential difference reaching its maximum 0.5 hour after the coating is perceived as compared with non-treated skin surface (control). On the other hand, it can be seen that, coating skin surface with capsaicin, followed by coating of capsazepine, the range of change in potential difference is reduced, and the change in potential difference is reduced as a result of the coated capsazepine inhibiting the skin surface stimulation by capsaicin.

Other than the above Examples, similar results were obtained when histamine, dodecylbenzene sulfate (surfactant), or lactic acid(organic acid) was used as a stimulating substance.

### (Example 5)

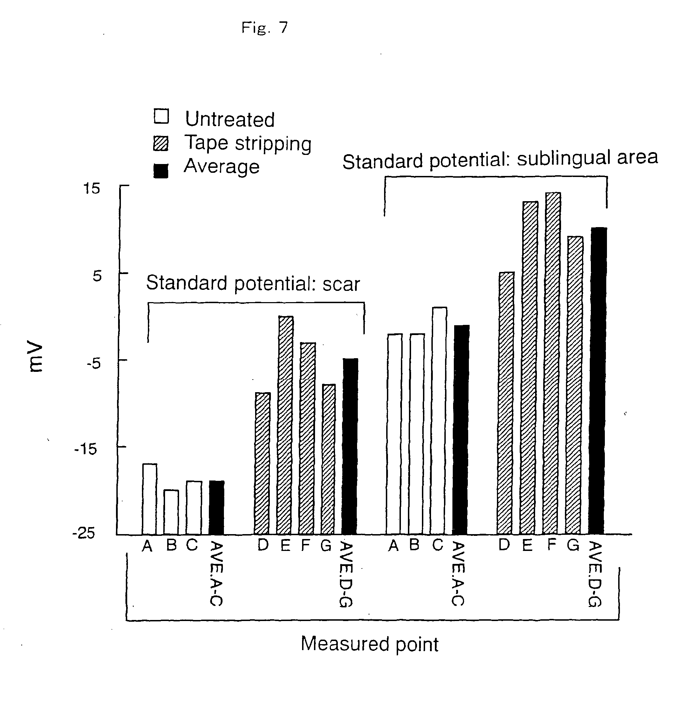

The potential differences of skin surface relative to a sublingual area and a scar on the skin (epidermis) were measured using the skin surface measuring apparatus shown in Fig. 2. The skin used in measurement is a skin in a state of 2 hours after skin surface was barrier-destructed (tape stripped) with a cellophane adhesive tape. Fig. 7 shows potentials at several places of skin using each of a sublingual potential and a skin scar potential as a standard potential.

As shown in Fig. 7, a potential rises 2 hours after tape stripping. It can be seen that this rise in potential is detectable using either a sublingual area or a skin scar as a standard. In addition, letters A to G in the figure denote measurement points, and AVE. denotes an average value.

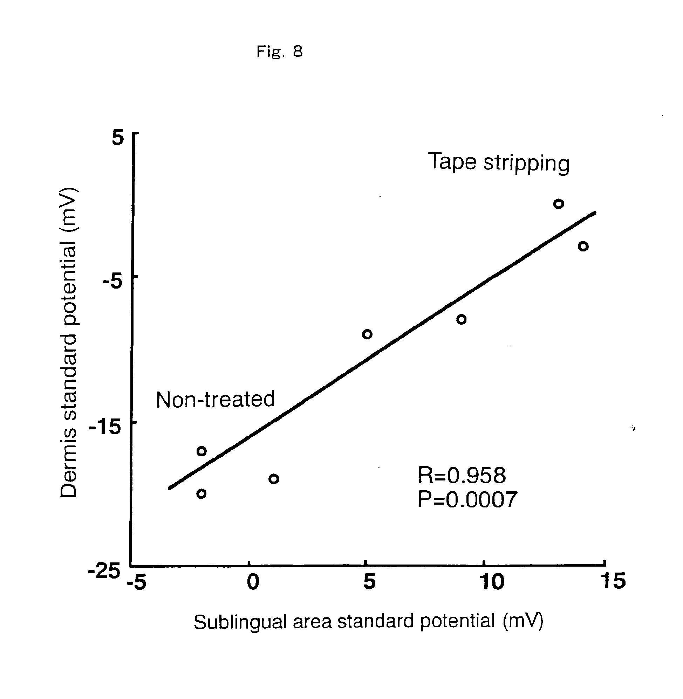

Furthermore, Fig. 8 plots the results of Fig. 7 in connection with the relationship between a skin surface potential using a skin scar as a standard and a skin surface potential using a sublingual area as a standard. It can be seen that there is significant correlation between both potentials, and also when a sublingual area is used as a standard in place of the previous method using a skin scar as a standard, a skin surface potential can be stably measured.

### Industrial Applicability

As explained above, according to the skin surface condition measuring apparatus of the present invention, by making a skin model, in which a skin section is floated with its surface upward on the surface of a culture solution, a potential difference between the skin surface and the culture solution can be measured simply. Furthermore, by adding a stimulating factor to the skin surface or a culture solution, a potential difference between the skin surface and a culture solution is increased, and as a result, the degree of stimulation given to skin can be confirmed. In addition, by coating a substance for warding off stimulation on skin, or by adding it to a culture solution, either before or after impartation of a stimulating factor to skin, or by adding a substance to ward off stimulation, the degree of a change in a potential difference is decreased, and an inhibiting substance to skin stimulation can be investigated.

Furthermore, according to the skin surface condition (potential condition) measuring apparatus, which uses a sublingual potential as a standard, the skin surface condition can be measured simply without imparting pain to a human. From this result, a number of useful applications can be expected such as confirmation of the degree of stimulation to be imparted to skin, investigation of an inhibiting substance to skin stimulation from a change in a potential difference by coating a substance for warding off stimulation on skin, and the like.

## Claims

1. A method for measuring the skin surface condition, which comprises: floating a skin section, with its surface upward, on the surface of a culture solution accommodated in a container; and measuring a potential difference between the skin surface of the skin section and said culture solution.

2. The method for measuring the skin surface condition according to claim 1, wherein said culture solution is a culture solution containing physiological saline or amino acids.

3. The method for measuring the skin surface condition according to claim 1 or 2, wherein the skin surface condition is a potential condition of the skin surface.

4. A method for measuring the degree of skin stimulation, which comprises: floating a skin section, with its surface upward, on the surface of a culture solution accommodated in a container; measuring a potential difference by imparting stimulation to the skin surface upon measuring of a potential difference between the skin surface of the skin section and the culture solution; and determining the degree of skin stimulation from a difference between the measured potential difference value and a potential difference when no stimulation is imparted.

5. The method for measuring the degree of skin stimulation according to claim 4, wherein said stimulation is made by a stimulating substance.

6. The method for measuring the degree of skin stimulation according to claim 4, wherein said stimulation is made by physical stimulation.

7. A method for measuring the degree of skin stimulation, which comprises: floating a skin section, with its surface upward, on the surface of a culture solution accommodated in a container; measuring a potential difference by adding a stimulating substance to a culture solution upon measuring a potential difference between the skin surface of the skin section and said culture solution; and determining the degree of skin stimulation from a difference between the measured potential difference value and a potential difference when no stimulating substance is added.

8. The method for measuring the degree of skin stimulation according to claim 5 or 7, wherein the said stimulating substance is a substance which generates an itch to skin.

9. The method for measuring the degree of skin stimulation according to any one of claims 4 through 8, wherein the said culture solution is a culture solution containing physiological saline or amino acids.

10. A method for measuring the degree of inhibition of skin stimulation by a drug, which comprises: floating a stimulated skin section, with its surface upward, on the surface of a culture solution accommodated in a container; measuring a potential difference by coating a drug on the skin surface, either before or after imparting stimulation to the skin surface upon measuring a potential difference between the skin surface of the skin section and said culture solution; and determining the degree of inhibition of skin stimulation by the drug from a difference between the measured potential difference value and a potential difference when no drug is coated.

11. A method for measuring the degree of inhibition of skin stimulation by a drug, which comprises: floating a stimulated skin section, with its surface upward, on the surface of a culture solution accommodated in a container; measuring a potential difference by adding a drug to the culture solution, either before or after imparting stimulation to the skin surface upon measuring a potential difference between the skin surface of the skin section and said culture solution; and determining the degree of inhibition of skin stimulation by the drug from a difference between the measured potential difference value and a potential difference when no drug is added.

12. The method for measuring the degree of inhibition of skin stimulation according to claim 10 or 11, wherein the said stimulation is made by a stimulating substance.

13. The method for measuring the degree of inhibition of skin stimulation according to claim 10 or 11, wherein the said stimulation is made by physical stimulation.

14. A method for measuring the degree of inhibition of skin stimulation by a drug, which comprises: floating a skin section, with its surface upward, on the surface of a culture solution accommodated in a container, with a stimulating substance added thereto; measuring a potential difference by coating a drug on the skin surface either before or after adding the stimulating substance to the culture solution upon measuring a potential difference between the skin surface of the skin section and said culture solution; and determining the degree of inhibition of skin stimulation by the drug from a difference between the measured potential difference value and a potential difference when no drug is coated.

15. A method for measuring the degree of skin stimulation by a drug, which comprises: floating a skin section, with its surface upward, on the surface of a culture solution accommodated in a container, with a stimulating substance added thereto; measuring a potential difference by adding a drug to the culture solution either before or after adding the stimulating substance to the culture solution upon measuring a potential difference between the skin surface of the skin section and said culture solution; and determining the degree of inhibition of skin stimulation by the drug from a difference between the measured potential difference value and a potential difference when no stimulating substance is added.

16. The method for measuring the degree of inhibition of skin stimulation according to claim 12, 14 or 15, wherein the said stimulating substance is a substance which generates an itch to skin.

17. The method for measuring the degree of inhibition of skin stimulation according to any one of claims 10 through 16, wherein the said culture solution is a culture solution containing physiological saline or amino acids.

18. The skin surface condition measuring apparatus, which comprises: a culture solution; a container accommodating a skin section floated with its surface upward on the surface of the culture solution; a standard electrode part communicating to said culture solution; a measuring electrode part communicating to the skin surface of said skin section; and a potential difference detecting part for detecting a potential difference between said standard electrode part and saidmeasuring electrode part.

19. The skin surface condition measuring apparatus according to claim 18, wherein the said standard electrode part is provided with an inner tube accommodating an internal solution with a standard electrode immersed therein, and an outer tube accommodating an internal solution which accommodates the inner tube to form a double tube, and a liquid junction is attached to the wall of said inner tube.

20. The skin surface condition measuring apparatus according to claim 19, wherein communication between the said culture solution and the standard electrode part is made by a salt bridge connecting said culture solution and the internal solution of said outer tube.

21. The skin surface condition measuring apparatus according to any one of claims 18 through 20, wherein the said measuring electrode part is provided with an inner tube accommodating an internal solution with a measuring electrode immersed therein, and an outer tube accommodating an internal solution which accommodates the inner tube to form a double tube, and a liquid junction is attached to the wall of said inner tube.

22. The skin surface condition measuring apparatus according to claim 21, wherein communication between the skin surface of said skin section and the measuring electrode part is made by a salt bridge connecting the skin surface of said skin section and the internal solution in said outer tube.

23. The skin surface condition measuring apparatus according to any one of claims 18 through 22, wherein the said culture solution is a culture solution containing physiological saline or amino acids.

24. The skin surface condition measuring apparatus according to any one of claims 18 through 23, wherein the skin surface condition is the potential condition of the skin surface.
